# EUROPÄISCHE PATENTANMELDUNG

(11) **EP 2 014 225 A2**
(43) Veröffentlichungstag der Anmeldung: **14.01.2009**
(21) Anmeldenummer: 08160156.9
(22) Anmeldetag: 10.07.2008
(51) Int. Cl.: A61B 5/00, G06F 19/00, A61B 5/0205

(54) **Verfahren zur Fernüberwachung des medizinischen Zustandes eines Nutzers, System und Vorrichtungen dafür**

(30) Priorität: 11.07.2007 DE 102007032610
(71) Anmelder: Deutsche Telekom AG, 53113 Bonn (DE)
(72) Erfinder: Neumann, Michael, 10777, Berlin (DE); Delloch, Manon, 13439, Berlin (DE)
(74) Vertreter: 2K Patentanwälte Kewitz & Kollegen

(57) **Zusammenfassung**

Es wird ein Verfahren zur Fernüberwachung des medizinischen Zustandes eines Nutzers mit folgenden Schritten vorgeschlagen: erste Daten (DAT1) werden durch mindestens einen am Körper des Nutzers angeordneten Sensor (S1,S2,S3...) erfasst (D1), anschließend werden die ersten Daten (DAT1) an eine Vorrichtung (SP) zum Auswerten der Daten übertragen (D2), anschließend werden die ersten Daten (DAT1) in der Vorrichtung (SP) ausgewertet (D3), wobei in Abhängigkeit von der Datenauswertung geprüft wird, ob Benachrichtigungen an Empfangsvorrichtungen (T1,T2,T3) gesendet werden, die mit der Vorrichtung (SP) über Kommunikationsnetzwerke (IP,UMTS) verbindbar sind (D4), und wobei in Abhängigkeit von der Datenauswertung auch bestimmt wird, welche der Empfangs-vorrichtung (T2) über welches der Kommunikationsnetzwerke (IP) und mit welcher Benachrichtigungsart benachrichtigt wird (D5). Es wird, die Datenauswertung von am Körper des Nutzers erfasster Sensordaten vorgeschlagen, wobei abhängig davon ggf.. eine gezielte Benachrichtigung von Empfangsvorrichtungen veranlasst wird, die beispielsweise bei einem Arzt oder einer anderen Vertrauenspersonen des Nutzers bzw. bei Institutionen (Rettungsdienst, Pflegedienst etc.) oder auch beim Nutzer selbst (Rückmeldung) vorgesehen sein können.

## Beschreibung

Die vorliegende Erfindung nimmt gem. Art. 87 u. 88 EPÜ die Priorität der früheren Deutschen Patentanmeldung DE 10 2007 032 610 in Anspruch, die am 11.07.2008 bei der zuständigen Behörde eingereicht wurde und deren Inhalt hiermit durch Bezugnahme in die vorliegende Anmeldung integriert ist.

Die Erfindung betrifft ein Verfahren zur Fernüberwachung des medizinischen Zustandes eines Nutzers sowie ein System und Vorrichtungen, insbesondere ein mobiles Endgerät, dafür.

Ein Verfahren und System zur Fernüberwachung des medizinischen Zustandes eines Nutzers ist z.B. aus der EP 1 710 766 A1 bekannt. Dort wird ein Mobilfunk-gestütztes System vorgeschlagen, bei dem biometrische Daten von einem am Körper des Nutzers befindlichen Sensor über ein damit verbundenes mobiles Endgerät erfasst werden. Im Fall von abnormalen Daten wird ein Signal an eine bestimmte Empfangsvorrichtungen bzw. Empfangsstation, nämlich an den Computer einer Kontaktperson ("prespecified contact address"), oder an eine Zentrale ("management center") übertragen. Dieses Verfahren und System erlauben also eine Benachrichtigung einer bestimmten Person oder Zentralstelle, falls abnormale Daten festgestellt werden. Dabei handelt es sich um medizinische oder allgemeine Notfälle. Wünschenswert ist es, darüber hinaus gehende Lösungen zu haben, die auch bei weniger dramatischen und/oder zumindest für den Nutzer selbst eher unkritischen Situationen eine oder mehrere Benachrichtigungen veranlassen, wobei die jeweilige Benachrichtigung situationsbedingt gesteuert wird, so dass nur relevante Benachrichtigungen an geeignete Empfänger erfolgen.

Weitere aus dem Stand der Technik zu nennende Dokumente sind die GB-A-2399204, DE-A-10130562, DE-A-102005006037, DE-A-102005006024, EP-A-11883997 und US-A-6064898.

Bei den bekannten Verfahren und Systemen hat sich gezeigt, dass sich Menschen bzw. einzelne Personen oft in vermeintlich unkritischen Situationen befinden, in denen sich ihre Emotionen ungünstig auf Ihre eigene Gesundheit und/oder unerwünscht auf ihre Umgebung sowie auf nachgelagerte Prozesse bzw. Systeme auswirken und somit ein Handlungsbedarf im mittel- bzw. unmittelbaren Umfeld der jeweiligen Person besteht. Ein solcher Handlungsbedarf besteht beispielsweise bei Kindern und älteren Menschen, insbesondere bei Krebspatienten, deren Gesundheitszustand auch von emotionalen Veränderungen abhängen, oder bei Menschen, die mit Systemen oder Geräten bzw. Maschinen arbeiten, deren fehlerhafte Bedienung aufgrund von Emotionen, wie beispielsweise Wut oder Stress, eine Gefahr oder zumindest eine unerwünschte Auswirkung für die Umgebung darstellt. In solchen Fällen ist es bedeutsam, schnell und direkt in bzw. auf die nachfolgenden Prozesse und Systeme einzugreifen.

Die Erfindung hat daher die Aufgabe, hier eine neue und besonders vorteilhafte Lösung zu den oben genannten Problemen vorzuschlagen. Insbesondere soll ein Verfahren zur Fernüberwachung des medizinischen und emotionalen Zustandes eines Nutzers vorgeschlagen werden, das in der Lage ist, nicht nur medizinisch relevante, sondern auch situationsbedingte Zustände, insbesondere emotionale Zustände, des Nutzers zu erkennen und intelligent die Benachrichtigung von geeigneten Empfängern zu steuern.

Gelöst wird die Aufgabe durch ein Verfahren mit den Merkmalen des Anspruchs 1.

Demnach wird ein Verfahren mit folgenden Schritten vorgeschlagen:
Es werden zumindest erste Daten [TI1]durch mindestens einen am Körper des Nutzers angeordneten Sensor erfasst;
Die ersten Daten werden anschließend an eine Vorrichtung zum Auswerten der Daten übertragen;
Und anschließend werden die ersten Daten in der Vorrichtung ausgewertet, wobei in Abhängigkeit von der Datenauswertung geprüft wird, ob Benachrichtigungen an Empfangsvorrichtungen gesendet werden, die mit der Vorrichtung über Kommunikationsnetzwerke verbindbar sind,
und wobei in Abhängigkeit von der Datenauswertung auch bestimmt wird, welche der Empfangsvorrichtung über welches der Kommunikationsnetzwerke und mit welcher Benachrichtigungsart benachrichtigt wird.

Außerdem werden auch ein das Verfahren ausführendes System zur Fernüberwachung des medizinischen und insbesondere psychologischen/emotionalen Zustandes eines Nutzers vorgeschlagen sowie ein dafür vorgesehenes mobiles Endgerät. Die Merkmale dazu sind in den nebengeordneten Ansprüchen formuliert.

Durch die Erfindung wird zur Fernüberwachung des medizinischen Zustandes eines Nutzers eine intelligente Datenauswertung von am Körper des Nutzers erfassten Sensordaten vorgeschlagen, wobei abhängig davon ggf.. eine gezielte Benachrichtigung von Empfangsvorrichtungen veranlasst wird, die beispielsweise bei einem Arzt oder einer anderen Vertrauensperson des Nutzers bzw. bei Institutionen (Rettungsdienst, Pflegedienst etc.) oder auch beim Nutzer selbst (Rückmeldung) vorgesehen sein können. Dabei wird auch die optimale Benachrichtigungsart bestimmt, wie z.B. Sprachnachricht (direkt und/oder auf VoiceBox), SMS und/oder Email usw.. Damit einhergehend werden vorzugsweise die übermittelten Inhalte der Nachricht bestimmt, wie z.B. Text, Bild, Video usw. und/oder die Datenformate.

Was die Sensorik und Datenerfassung angeht, so stellt die Einbeziehung von Emotionen in die Messung und Auswertung eine zusätzliche Komponente dar, um einerseits den spezifischen Gefühlszustandes einer Person beispielsweise in der Gestaltung der Benachrichtigung zu berücksichtigen und andererseits auch spezifische Situationen und möglicherweise daraus resultierende Gefühlsschwankungen zu erkennen.

Mit der Erfindung können über Sensoren, insbesondere biometrische Sensoren, als erste und/oder zweite Daten erfasst und somit insbesondere auch die Emotionen des Nutzers erkannt werden. Die Daten können gegebenenfalls gespeichert werden und können unabhängig von einem bestimmten Verfahren oder Software ausgewertet werden. Auf Basis der Auswertungsergebnisse können dann die sich daran anschließenden Prozesse und Systeme bzw. Geräte teil- oder vollautomatisiert gesteuert werden.

Dies kann insbesondere bedeuten, dass eine Empfangsvorrichtung bzw. Empfangsstation (z.B. Notarzt oder Notrufsystem) aktiviert wird, welche eine Benachrichtigung an eine definierte Person bzw. Personengruppe (z.B. Eltern, Arzt, Polizei) ausgibt, oder dass ein Gerät aktiviert wird, welches anhand zu definierender Parameter eine entsprechende Leistung erhöht oder verringert. Denkbar ist auch, dass ein Prozess, System oder Gerät deaktiviert und damit beendet bzw. abgeschaltet wird.

Die Erfindung kann insbesondere zur Daten-Erfassung via Telekommunikationsnetze und zur (teil-)automatisierten Auswertung der mittels Sensorik gemessenen emotionalen Zustände eingesetzt werden. Auch kann sie für die (teil-) automatisierte Steuerung nachfolgender Prozesse und Systeme dienen, insbesondere durch Initiierung von Signalen, beispielsweise im Format von Nachrichten bzw. Benachrichtigungen, sowie zur telekommunikativen Weiterleitung und damit gegebenenfalls Aktivierung/Deaktivierung von Systemen und Geräten auf der Empfängerseite dienen.

Vorzugsweise sind die Sensoren bzw. eine dafür eingerichtete Messvorrichtung in dem mobilen Endgerät integriert.

Auch werden vorzugsweise folgende Daten erfasst und ausgewertet:
Zum einen sind es erste Daten, die allgemeine biomedizinische Parameter, insbesondere Bio-Feedback-Parameter wie Feuchtigkeit und Hautspannung, betreffen, aus denen der allgemeine medizinische Zustand des Nutzers auswertbar ist. Zum anderen sind es auch zweite Daten, die spezielle biomedizinische Parameter, insbesondere Blutwert- und/oder Nierenwert-Parameter, betreffen, aus denen ein Stresszustand des Nutzers auswertbar ist. Dadurch ergibt sich eine sehr vorteilhafte kombinierte Datenauswertung, die es erlaubt, mit hoher Wahrscheinlichkeit eine korrekte Ferndiagnose über den medizinischen, insbesondere emotionellen Zustand des Nutzers zu stellen.

Im Vergleich zu herkömmlichen Lösungen, bei den sog. Biofeedback-Verfahren angewendet werden, um beispielsweise Hautspannung oder Feuchtigkeit zu messen und somit auf einen Stresszustand zu schließen, zielt die Erfindung auf eine Kombination von Messparametern ab, welche sowohl Biofeedback-Parameter wie auch weitere Parameter, insbesondere Blutwerte und adäquate Parameter, auswertet. Hierzu sind insbesondere so genannte Nierenwerte und Stresshormone zu nennen, anhand derer der Stresszustand im Zusammenhang mit den gemessenen Biofeedback-Daten festgestellt werden kann. Dies erfolgt vorzugsweise innerhalb des Endgerätes. Die Auswertung der Daten kann aber auch in der Dienstezentrale oder an anderer zentraler Stelle erfolgen. Zudem kann auch eine teilautomatisierte Auswertung stattfinden. Das erfindungsgemäße System kann als Multi-Client-System ausgebaut werden, welches ein intelligentes Routing bzw. Forwarding der Benachrichtigungen bzw. Notrufe steuert. Somit gehen die Benachrichtigungen bzw. Notrufe wahlweise an eine Zentrale, den Arzt, Eltern, oder Freunde etc.. Die Empfänger werden also selektiv und gezielt sowie über optimale Kommunikationsverbindungen in einer passenden Form benachrichtigt.

Wenn z. B. ein kindlicher Krebspatient zwar physisch keine kritische Situation nachweisen lässt, aber Angst vor einer anstehenden Untersuchung hat, so wirkt sich das auf die folgende Untersuchung ggf. negativ aus (z. B. Bluthochdruck, Verweigerunghaltung...), obwohl die Eltern die Situation durch Beruhigung des Kindes weitgehend entschärfen könnten, wenn sie denn davon wüßten. In beispielsweise solchen Situationen kann die Erfindung in vorteilhafter Weise Abhilfe schaffen.

Diese und weitere besonders bevorzugte Ausgestaltungen der Erfindung werden durch die Unteransprüche angegeben.

Insbesondere werden gemäß der Erfindung, verschiedene Parameter (Biofeedback und Blutwerte) ausgewertet und anhand der Auswertung eine Steuerung der Nachrichtenübertragung (intelligentes Routing bzw. Forwarding) veranlasst.

Zwar gibt es bereits verschiedene Systeme mit emotionaler Sensorik, um Emotionen zu erkennen. Zu nennen sind hier unter anderem Blutdrucksensoren (Blood Volume Pulse), Hautwiderstandssensoren (Galvanic Skin Response), Sensoren zur Messung von Muskelaktivitäten (Electromyograph) sowie Sensoren zur Messung von Atemgeschwindigkeit und Volumen (Respiration Sensor). Die Signale werden aber dort durch einen Analog-Digital-Wandler in digitale Signale umgewandelt und dann zu einem Computer weitergeleitet, um dort weiterverarbeitet zu werden. Dieser Computer ist jedoch in der Regel direkt an den Sensor gegebenenfalls via Kabel gekoppelt. Eine Übertragung der Daten via Telekommunikationsnetz findet nicht statt.

Auch ergänzen so genannte "Umgebungssensoren" zur Erkennung menschlicher Verhaltensmuster die Interpretationsmöglichkeiten menschlicher Stimmungen und sichern damit die Evidenz von aussagekräftigen Rückschlüssen auf die Emotionen eines Menschen.

In dem erfindungsgemäßen Verfahren werden diese Sensoren sowie die von den Sensoren gemessenen Signale kombinatorisch genutzt.

Im Unterschied dazu beziehen sich bekante Verfahren und Vorrichtungen zum Abfragen und Speichern von Vitalparametern, wie z.B. die Herzfrequenz, schwerpunktmäßig auf die Sensorik selbst, sprich die Aufnahme von Daten bzw. Signalen und ggf. Speicherung im Sensor bzw. im direkt angeschlossenen Speichermedium. In einigen Fällen sind daher Speichermedien zur Aufbewahrung der Daten im Sensor integriert. Eine automatisierte Verwendung der Daten, im Sinne einer Auswertung und darauf basierender Initiierung und Steuerung nachgelagerter Prozesse und Systeme bzw. Geräte, findet in den bekannten Systemen jedoch nicht statt. Ebenso nicht eine telekommunikative Kopplung an ein Notrufsystem.

Die üblichen Anwendungen von emotionaler Sensorik liegen vor allem in den Bereichen Wellness/Fitness, der Fahrzeugindustrie und der Spieleindustrie - im letzteren Fall wird teilweise mittels Emotionen Einfluss auf konkrete Spielzüge oder den weiteren Inhalt innerhalb eines Spieles beschrieben. Der Medizinbereich ist auf die sensorische Steuerung von Herzschrittmachern fokussiert. In keinem der üblichen Lösungen findet eine Übertragung von Daten über das Telekommunikationsnetz statt. In den Bereichen für die Erkennung von Emotionen in Texten und Sprache geht es primär um die Identifizierung von Emotionen in diversen Medien, jedoch nicht um eine automatisierte Einflussnahme auf nachgelagerte Prozesse und Systeme, insbesondere nicht im Sinne telekommunikativer Weitergabe von Daten, Aktivierung/ Deaktivierung von Systemen und Geräten auf Basis von Signalen und sonstigen Daten, etwa im Sinne eines Notrufsystems.

Allen bisher bekannten Systemen ist gemeinsam, dass Emotionsparameter lediglich erkannt und angezeigt werden. Die Erfindung geht weit darüber hinaus und schlägt eine Weiterleitung und gegebenenfalls Speicherung von emotionalen Daten mit Hilfe von IT-/TK-Technologien sowie die (teil-) automatisierte Auswertung vor sowie eine darauf folgende Indizierung von Folgeaktivitäten, insbesondere in Form der Aktivierung/Deaktivierung von Systemen bzw. Geräten wie auch die Versendung von Benachrichtigungen bzw. Notrufsignalen, beispielsweise in Form einer Benachrichtigungs-SMS oder dergleichen.

Eine weitere Besonderheit der Erfindung besteht in der für die Steuerung nachgelagerter Prozesse und Systeme bzw. Geräte relevanten (teil-)automatisierten Auswertung von Vitalparametern. Letztere werden von der hier vorgeschlagenen Lösung in ihrer Art erkannt und entsprechend der identifizierten Signale klassifiziert, bewertet und aufgezeichnet.

Die Auswertung selbst orientiert sich vorzugsweise an medizinischen Normwerten und Abweichungen hinsichtlich der individuellen Aufzeichnungsdaten. Eine Speicherung der Daten ermöglicht den sicheren und personalisierten Zugang für autorisierte Personen einerseits via Web-Schnittstelle oder VPN (Virtuelles Privates Netz) sowie andererseits via direkter, verschlüsselter Weiterleitung von Daten bzw. Ergebnisse der Auswertung über Telekommunikationsnetze, wie beispielsweise UMTS/ GPRS/DSL-Kanäle.

Nachfolgend wird die Erfindung nun anhand eines Ausführungsbeispiels im Detail näher beschrieben, wobei auf die beiliegenden Zeichnungen Bezug genommen wird, welche folgende schematische Darstellungen wiedergeben:

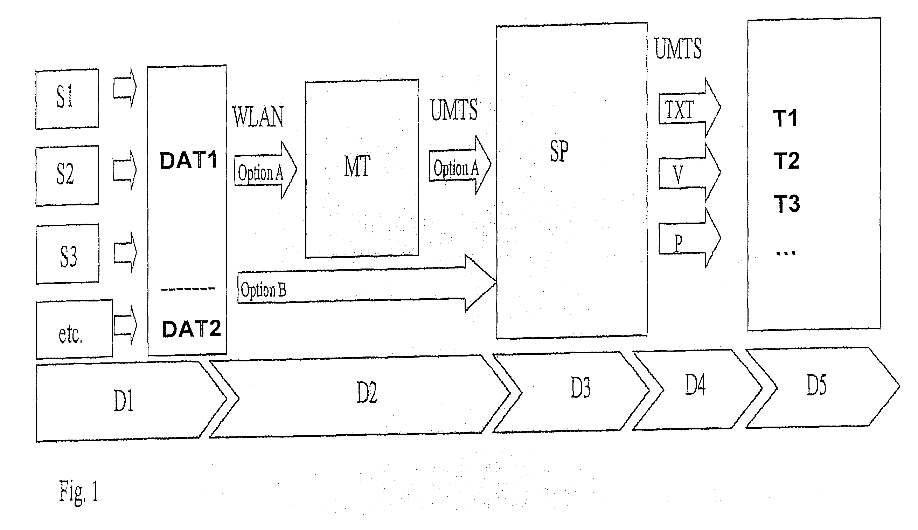

- Fig. 1: zeigt anhand von schematischen Funktionsblöcken den Ablauf eines erfindungsgemäßen Verfahrens; und

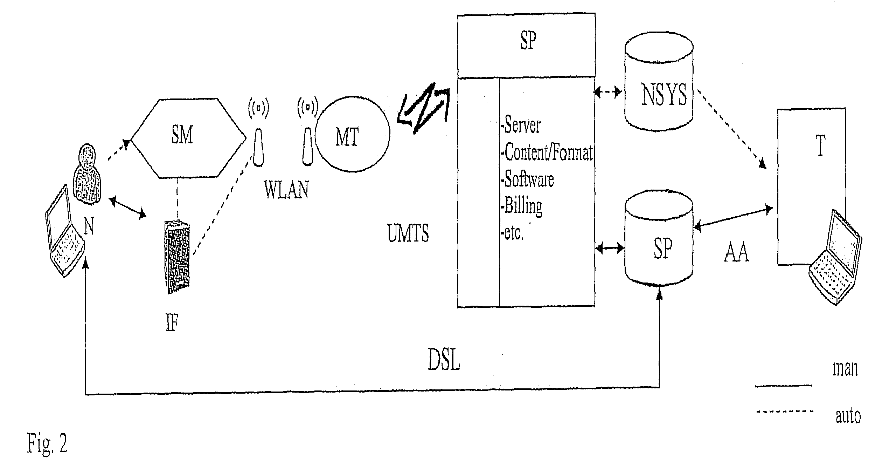

- Fig. 2: zeigt schematisch den Aufbau eines erfindungsgemäßen Systems.

In der folgenden Beschreibung wird auf beide Figuren Bezug genommen:

Das Verfahren zur Fernüberwachung des medizinischen Zustandes eines Nutzers N beginnt damit, dass in einem Schritt D1 erste Daten DAT1 durch mindestens einen am Körper des Nutzers N angeordneten Sensor, hier z.B. durch die Sensoren S1, S2 und S3 erfasst werden. Der Nutzer N trägt ein mobiles Endgerät MT, an dem die Sensoren S1, S2, und S3 angeschlossen sind bzw. mit dem die Sensoren in kommunikativer Verbindung stehen, um erste Daten DAT1 in Form von biomedizinischen Parametern, insbesondere Bio-Feedback-Parametem, zu erfassen und weiterzuleiten, aus denen sich auf den allgemeinen medizinischen Zustand des Nutzers N schließen lässt. Die Sensoren selbst und/oder das Endgerät MT können eine Speicherfunktion SM aufweisen, um die erfassten Daten für eine nachfolgende Auswertung oder Übertragung zwischenzuspeichern. Die Speicherfunktion SM kann auch als Speicherkarte ausgebildet sein, die in das Endgerät MT oder einen beliebigen PC eingesteckt werden kann. Die Sensoren können z.B. per Kabel an das mobile Endgerät MT angeschlossen sein oder auch darin integriert sein. Neben den ersten Daten DAT1 werden auch noch zweite Daten DAT2 erfasst, die zweite biomedizinische Parameter, insbesondere Blutwert- und/oder Nierenwert-Parameter (z.B. Nebennierensteroide), betreffen, aus denen ein spezifischer Zustand des Nutzers N auswertbar ist. Diese zweiten Daten dienen dazu, den spezifischen Zustand zu eruieren, welcher von der Grunderkrankung des Patienten abhängig ist bzw. von der Situation, in der sich der Patient befindet (z.B. Kind im Krankenhaus vor einer OP oder Seniorin in einem Pflegeheim.

Die erfassten Daten DAT1 und DAT2 werden dann in einem nächsten Schritt D2 an eine Vorrichtung zum Auswerten der Daten übertragen, wobei diese Vorrichtung das Endgerät MT und/oder eine damit verbundene Dienstezentrale SP sein kann. Die Daten werden also entsprechend an das mobile Endgerät MT übertragen und dann weiter an die Dienstezentrale SP übertragen (Option A) oder werden direkt an die Dienstezentrale SP übertragen (Option B). Dabei erfolgt die Übertragung von den Sensoren S1, S2 und S3 bzw. der Speicherfunktion SM (Speicherkarte) an das mobile Endgerät MT beispielsweise über ein lokales Funknetz WLAN. Die weitere Übertragung an die Dienstezentrale SP erfolgt beispielsweise über ein öffentliches Mobilfunknetz UMTS. Andere IT/TK-Übertragungswege sind ebenfalls denkbar. Die Übertragung kann in irgendeiner beliebigen Weise, z.B. via Kabel, via TK-Technologien, wie z.B. Bluetooth, oder integriert erfolgen.

Die Dienstezentrale SP hat einen Server und ist als Server-Pattform ausgebildet, auf die über entsprechende Clients beispielsweise per HTTP-Protokoll bzw. per Browser zugegriffen werden kann. Die Clients sind auf Rechnern bzw. PCs von Ärzten, Notfalldiensten, Pflegediensten usw. oder auch von Privatpersonen installiert, welche ggf. benachrichtigt werden sollen bzw. müssen. Diese Rechner bzw. PC bilden somit Empfangsvorrichtungen T1, T2 und T3 für die evtl. dort eingehenden Benachrichtigungen. Für die Verbindung zwischen Dienstezentrale SP und Empfangsvorrichtungen bzw. Empfangsstationen T1, T2, T3 usw. stehen ebenfalls verschiedene Kommunikationsnetzwerke zur Verfügung, wie z.B. terrestrische Festnetze oder Mobilfunknetze oder auch satelliten-gestütze Netze und Kombinationen daraus. Vorzugsweise erfolgt die Kommunikation im Rahmen einer Internet-Verbindung IP, die über mindestens eines der Netze aufgebaut wird. Zur Benachrichtigung einzelner Empfangsvorrichtungen, z.B. per Kurznachricht (SMS; MMS, Voice-Mail/Sprachnachricht), kann aber auch eine andere Verbindung, z.B. eine Mobilfunkverbindung UMTS, aufgebaut werden.

Um nun die Benachrichtigungen intelligent zu steuern, wertet die Dienstezentrale SP in einem Schritt D3 die erfassten und an sie übertragenen Daten DAT1 und DAT2 aus und bestimmt, ob überhaupt ein Benachrichtigungsfall eingetreten ist, und welche der in Frage kommenden Empfangsvorrichtungen bzw. Empfangsstationen eine bzw. welche Benachrichtigung erhalten sollen, und über welche Kommunikationsverbindung bzw. welches Kommunikationsnetz die Benachrichtigung in welcher Form ausgesendet werden soll. So wird z.B. ein mobiler Empfänger über eine entsprechende Mobilfunkverbindung UMTS benachrichtigt, wobei noch bestimmt wird, in welcher Form dies geschieht, also z.B. in Textform TXT als Textnachricht bzw. Kurznachricht. Andere Empfänger werden z.B. über eine breitbandige Internet- bzw. DSL-Verbindung multimedial benachrichtigt, wobei die Informationen auch im Videoformat V und/oder Bilddatenformat P übertragen werden. Die Übertragung der Daten bzw. Informationen erfolgt in einem Schritt D4 und die Benachrichtigung in einem Schritt D5, z.B. durch optische und/oder akustische Anzeige an der jeweiligen Empfangsvorrichtung. Beispiel für eine solche Benachrichtigung könnte ein täglicher Statusbericht von Frau Müller sein mit dem Hinweis "Keine besonderen Anzeichen von Erregung identifizierbar. Oder es könnte eine Benachrichtigung an Eltern sein, wie z.B. "Ihr Sohn Marcel ist zur Zeit sehr aufgeregt. Bitte rufen Sie in der Klinik an."

In dem hier gezeigten Beispiel erfolgt die Auswertung der Daten DAT1 und DAT2 innerhalb der Dienstezentrale SP. Es kann aber auch vorteilhaft sein, wenn die Daten vor Ort beim Nutzer N, d.h. innerhalb des mobilen Endgerät MT, ganz oder zumindest zum Teil ausgewertet werden.

Auch ist denkbar, dass im Endgerät MT aus den erfassten Daten DAT1 und/oder DAT2 abgeleitete Daten gebildet werden, also eine Daten-Vorauswertung durchgeführt wird, und dass die abgeleiteten Daten dann zur weiteren Auswertung an die Dienstezentrale SP übertragen werden. Die Dienstezentrale SP ist vorzugsweise als internet-basierter Server eingerichtet, auf den die Empfangsvorrichtungen T1, T2, T3 usw. als Client Zugriff haben, so dass ein Multi-Client-System ausgebildet wird.

Das Endgerät MT kann als Mobilfunktelefon mit Zusatzfunktion oder als spezielles Mobilgerät (Vital-Messgerät) ausgebildet sein. Die Sensoren S1, S2, und S3 befinden sich beispielsweise in der Kleidung oder an Schmuckstücken (Armband, Ringe, etc.) und messen spezielle Vital- und Stressparameter, wie z.B. Hautwiderstand, Schweiß, Pulsfrequenz usw..

Die erfassten Daten DAT1 und/oder DAT2 werden je nach Ausstattung der Sensoren entweder mittels Funktechnologien (z.B. UMTS, WAP, etc.), Kabel oder ähnlichem, direkt oder indirekt über das Endgerät MT, das den Zugang zum Telekommmikationsnetz gewährleistet, an die Dienstezentrale bzw. Service Plattform SP gesendet, auf der die Daten empfangen, ggf. mittels Datenträger gespeichert und (teil-) automatisiert mittels Regelwerk und/oder Software klassifiziert und ausgewertet werden. Zudem ist es gegebenenfalls mittels Web-Interface oder VPN-Zugang möglich, sowohl gemessene Daten als auch die Klassifizierung und automatisiert erstellte Analyseergebnisse durch fachkundige Experten einzusehen, zu evaluieren und durch manuelle Bewertungen zu ergänzen. Damit ist gemeint, dass die die erfassten Daten automatisiert, beispielsweise anhand von Regeln ausgewertet und klassifiziert, werden.

Die Bereitstellung und gegebenenfalls Weiterleitung der Analyseergebnisse erfolgt ebenfalls über Telekommunikationsnetze, wie z.B. UMTS, ISDN, DSL, in Abhängigkeit der Datenformate sowie auf Basis eines definierten Regelwerkes, beispielsweise bei Abweichung von vorab definierten und eingestellten Normwerten. Widerspricht also das Ergebnis der Auswertung den vorher definierten und in Datenbanken gespeicherten Normwerten und/oder den sonst gemessenen Werten der beobachteten Person, so wird ein Dritter oder mehrere Personen oder Institution, wie z.B. Eltern, Ärzte bzw. sonstige Vertrauenspersonen via SMS, Sprachnachricht o.ä. informiert.

Darüber hinaus erfolgt eine Steuerung nachgelagerter Prozesse und Systeme bzw. Geräte in der Form, dass diese anhand definierter Parameter aktiviert bzw. deaktiviert und/ oder in ihrer Ausprägung beeinflusst werden, beispielsweise in der Form, dass Daten an ein sonstiges Speichermedien (z.B. digitale Patientenakte) weitergeleitet werden, ein Notfallsystem aktiviert wird und/ oder ein Gerät/ Maschine in ihrer Leistung gemindert bzw. erhöht wird.

Bei der beschriebenen Service-Plattform SP handelt es sich also um eine technologische Lösung, mit dessen Hilfe Daten in verschiedenen Formaten (Bild, Sprache, Text) erfasst, ggf. gespeichert, ausgegeben und/oder im Sinne einer Auswertung weiterverarbeitet werden können. Die technische Lösung kann hierbei sowohl in der Festlegung der Art der Auswertung, der zu benachrichtigenden bzw. informierenden Person bzw. Institution, der auszulösenden bzw. zu steuernden Prozesse als auch in der Art und Inhalt der Übertragung (Text, Sprache, Bild) fest definiert als auch individuell programmierbar sein. Hierzu können Eingabemöglichkeiten am Ort des Sensors oder auch separat über eine Telekommunikationsschnittstelle z.B. Web-Interface zur Service-Plattform geschaffen werden. So können etwa Eingabemöglichkeiten unter anderem durch den Nutzer oder Pflegepersonal geschaffen werden, beispielsweise als Rufnumerneingabe für eine Benachrichtigung. Um die Daten auf der Service Plattform unabhängig von Vorgang des Sensorischen Erfassens von Vitalparametern abzurufen, kann ein zusätzlicher Zugang zur technologischen Lösung eingerichtet werden, welcher in der Regel mit sicherheitsrelevanten Funktionen ausgestattet wird.

In der Praxis integriert die Gesamtlösung ein intelligentes Fernüberwachungs- und Benachrichtigungssystem, insbesondere ein technisches Notrufsystem, das im Rahmen eines einmaligen oder mehrmaligen Monitoring emotionale Veränderungsparameter erfasst, ggf. auswertet und mit Hilfe dieser Daten Nachfolgeprozesse und ggf Systeme initiiert sowie insbesondere steuert.

Die Kopplung der technischen Lösung an weitere (Dritt-) Systeme, bei denen unter anderem durch Datensignale oder Sprache die Funktion desselben aktiviert bzw. deaktiviert oder erhöht bzw. vermindert wird oder eine beliebige Person/Institution informiert wird, stellt eine Erweiterung des Systems dar, die ebenfalls der Lösung der Erfindung obliegt.

Verschiedenste Einsatzgebiete sind denkbar. So kann dies sowohl im Krankenhaus sein, beispielsweise wie oben bereits erwähnt bei der Betreuung von Kindern, deren Gesundheitszustand auch von emotionalen Veränderungen abhängen (z.B. Krebspatienten). Dies kann auch in Umgebungen sein, in denen Eltern einen Informationsbedarf hinsichtlich emotionaler Veränderungen ihrer Kinder, (Groß-)Eltern, hilfsbedürftigen Freunden etc. sehen. Denkbar ist darüber hinaus auch die Anwendung in Fahrzeugen oder bei der Handhabung von Maschinen, deren Gefahrenpotential durch unkontrollierbare Emotionen mit Hilfe der Lösung herabgesetzt werden kann.

Bezugszeichenliste
- D1-D5: Einzelne Verfahrensschritte
- D1: Daten-Erfassung über Sensoren SM bzw. S1-S3 am Körper des Nutzers N
- D2: Daten-Übertragung von Sensoren SM an Mobiles Endgerät MT und anschließend an zentrale Service Plattform SP
- D3: Daten-Auswertung und -Speicherung in Service Plattform SP
- D4: Daten-Übertragung von Service Plattform SP an Empfangsvorrichtungen T
- D5: Benachrichtigung einzelner Empfänger (Arzt, Eltern, Freunde)
- DAT1: Erste Daten, die Bio-Feedback-Parameter betreffen (Daten zum allg. Zustand)
- DAT2: Zweite Daten, die Blutwert- und/oder Nierenwert-Parameter betreffen (Stressdaten)
- S1-S3: Einzelsensor, z.B. an Kleidung, Armband, Halskette
- IF: Im Sensor integrierte Schnittstelle für Ein-/Ausgabe von Daten
- WLAN: Lokale Datenübertragung (z.B. über Wireless LAN, Bluetooth, Infrarot)
- MT: Mobiles Endgerät (z.B. Mobilkommunikationsgerät nach GSM/UMTS)
- UMTS: Mobile Datenübertragung (z.B. über UMTS, GPRS, WAP)
- SP: Dienstezentrale als Vorrichtung zur Datenauswertung und Service Plattform, insbesondere mit Server
- DB: Datenbank der Service Plattform SP
- SPT: Service Portal
- NSYS: Notruf-System
- AA: Autorisierter Zugang zum Service Portal SPT
- T1, T2, T3: Empfangsvorrichtungen bzw. Endgeräte bei Arzt, Eltern, Freunden
- IP: Kommunikationsverbindung (z.B. Internetverbindung) für Zugriff des Nutzers N auf Service Portal SP
- man: manuell veranlasste Kommunikation bzw. Datenübertragung
- auto: automatisch veranlasste Kommunikation bzw. Datenübertragung
- Option A: Datenübertragung über das Mobile Endgerät MT
- Option B: Direkte Datenübertragung zwischen Sensore SM und Service Plattform SP
- TXT, V, P: multimediale Dateninhalte bzw. -formate als Text, Video bzw. Bild

## Patentansprüche

1. Verfahren zur Fernüberwachung des medizinischen Zustandes eines Nutzers (N) mit folgenden Schritten:
zumindest erste Daten (DAT1) werden durch mindestens einen am Körper des Nutzers (N) angeordneten Sensor (S1, S2, S3...) erfasst (Schritt D1);
anschließend werden die ersten Daten (DAT1) an eine Vorrichtung (SP) zum Auswerten der Daten übertragen (Schritt D2; OptionB);
anschließend werden die ersten Daten (DAT1) in der Vorrichtung (SP) ausgewertet (Schritt D3), wobei in Abhängigkeit von der Datenauswertung geprüft wird, ob Benachrichtigungen an Empfangsvorrichtungen (T1, T2, T3) gesendet werden, die mit der Vorrichtung (SP) über Kommunikationsnetzwerke (IP, UMTS) verbindbar sind (Folgeschritt D4), und wobei in Abhängigkeit von der Datenauswertung auch bestimmt wird, welche der Empfangsvorrichtung (T2) über welches der Kommunikationsnetzwerke (IP) und mit welcher Benachrichtigungsart benachrichtigt wird (Folgeschritt D5).

2. Verfahren nach Anspruch 1, wobei die Vorrichtung zum Übertragen und/oder Auswerten der Daten ein mit den Sensoren verbundenes und von dem Nutzer (N) getragenes mobiles Endgerät (MT) ist, wobei die ersten Daten (DAT1) zumindest auch zum Teil durch das mobile Endgerät (MT) ausgewertet werden können.

3. Verfahren nach Anspruch 1, wobei die Vorrichtung zum Auswerten der Daten eine Dienstezentrale (SP) ist, die mit einem mit den Sensoren verbundenen und von dem Nutzer (N) getragenen mobilen Endgerät (MT) verbindbar ist, wobei die ersten Daten (DAT1) zumindest zum Teil durch die Dienstezentrale (SP) ausgewertet werden.

4. Verfahren nach einem der vorhergehenden Ansprüche, wobei ein mit den Sensoren verbundenes und von dem Nutzer (N) getragenes mobiles Endgerät (MT) die ersten Daten (DAT1) und/oder davon abgeleitete Daten an eine Dienstezentrale (SP) überträgt, von der ersten Daten (DAT1) ausgewertet werden (Schritt D3) bzw. die daraus abgeleiteten Daten weiter ausgewertet werden.

5. Verfahren nach Anspruch 1, wobei
die ersten Daten (DAT1) biomedizinische Parameter, insbesondere Bio-Feedback-Parameter, betreffen, aus denen der allgemeine medizinische Zustand des Nutzers (N) auswertbar ist.

6. Verfahren nach Anspruch 5, wobei
auch zweite Daten (DAT2) erfasst und ausgewertet werden, die spezielle biomedizinische Parameter, insbesondere Blutwert- und/oder Nierenwert-Parameter, betreffen, aus denen spezifische Zustände, entsprechend der vorher definierten Krankheitsbilder und/oder Monitoringdaten, des Nutzers (N) auswertbar ist.

7. Verfahren nach einem der vorhergehenden Ansprüche, wobei
in Abhängigkeit von der Auswertung der ersten Daten (DAT1) und der zweiten Daten (DAT2) geprüft wird, ob die Benachrichtigungen an Empfangsvorrichtungen (T1, T2, T3) gesendet werden (Teilschritt D4), und auch bestimmt wird, welche einzelne Empfangsvorrichtung (T2) über welches der Kommunikationsnetzwerke (IP) benachrichtigt wird (Teilschritt D5).

8. Verfahren nach einem der vorhergehenden Ansprüche, wobei
die Auswertung der Daten (DAT1; DAT2) zumindest teilweise automatisch von dem mobilen Endgerät (MT) und/oder von der Dienstezentrale (SP) ausgeführt wird.

9. System zur Fernüberwachung des medizinischen Zustandes eines Nutzers (N), mit folgenden Komponenten:
ein von dem Nutzer (N) getragenes mobiles Endgerät (MT), das mit mindestens einem am Körper des Nutzers (N) angeordneten Sensor (S1, S2, S3...) verbunden ist und damit gemessene erste Daten (DAT1) erfasst;
eine Dienstezentrale (SP), die mit dem Endgerät (MT) verbindbar ist und von diesem die ersten Daten (DAT1) und/oder davon abgeleitete Daten empfängt; Empfangsvorrichtungen (T1, T2, T3), die mit der Dienstezentrale (SP) über Kommunikationsnetzwerke (IP, UMTS, etc.) verbindbar sind und die in Abhängigkeit von einer Auswertung der ersten Daten (DAT1) und/oder der davon abgeleiteten Daten Benachrichtigungen empfangen, wobei die Dienstezentrale (SP) prüft, ob die Benachrichtigungen an die Empfangsvorrichtung (T1, T2, T3) gesendet werden, und in Abhängigkeit von der Datenauswertung auch bestimmt, welche der Empfangsvorrichtungen (T2) über welches der Kommunikationsnetzwerke (IP) und
mit welcher Benachrichtigungsart benachrichtigt wird.

10. System nach Anspruch 9, wobei der mindestens eine Sensor (S1) als die ersten (DAT1) biomedizinische Parameter, insbesondere Bio-Feedback-Parameter, misst, aus denen der allgemeine medizinische Zustand des Nutzers (N) auswertbar ist.

11. System nach Anspruch 10, wobei der mindestens eine Sensor und/oder das Endgerät zudem zweite Daten (DAT2) erfasst, die spezielle biomedizinische Parameter, insbesondere Blutwert- und/oder Nierenwert-Parameter, betreffen, aus denen ein spezifische Zustand, entsprechend der vorher definierten Krankheitsbilder und/oder Monitoringdaten, des Nutzers (N) auswertbar ist.

12. System nach einem der Ansprüche 9 bis 11, wobei
die Dienstezentrale (SP) als internet-basierter Server eingerichtet ist, auf den die Empfangsvorrichtung (T1, T2, T3) als Client Zugriff haben, so dass ein Multi-Client-System ausgebildet wird.

13. Mobiles Endgerät (MT) für ein System nach Anspruch 9, wobei
das mobile Endgerät (MT) von einem Nutzer (N) tragbar ist und mit mindestens einem am Körper des Nutzers (N) angeordneten Sensor (S1, S2, S3...) verbunden ist und damit gemessene erste Daten (DAT1) erfasst;
und wobei das Endgerät die ersten Daten (DAT1) insbesondere zum Teil oder ganz auswertet, die ersten Daten (DAT1) und/oder davon abgeleitete Daten an eine Dienstezentrale (SP) zur Benachrichtigungen von Empfangsvorrichtung (T1, T2, T3) sendet, die mit der Dienstezentrale (SP) über Kommunikationsnetzwerke (IP, UMTS.) verbindbar sind.

14. Mobiles Endgerät (MT) nach Anspruch 13, wobei der mindestens eine Sensor (S1) in dem Endgerät (MT) integriert ist, und wobei der mindestens eine Sensor (S 1) als die ersten (DAT1) biomedizinische Parameter, insbesondere Bio-Feedback-Parameter, misst, aus denen der allgemeine medizinische Zustand des Nutzers (N) auswertbar ist.

15. Mobiles Endgerät (MT) nach Anspruch 14, wobei das Endgerät auch zweite Daten (DAT2) erfasst, die spezielle biomedizinische Parameter, insbesondere Blutwert-und/oder Nierenwert-Parameter, betreffen, aus denen ein Stresszustand des Nutzers (N) auswertbar ist.
